# EUROPÄISCHE PATENTSCHRIFT

(11) **EP 1 735 006 B1**
(45) Veröffentlichungstag und Bekanntmachung des Hinweises auf die Patenterteilung: **26.09.2007**
(21) Anmeldenummer: 05716516.9
(22) Anmeldetag: 02.04.2005
(51) Int. Cl.: A61K 47/32, A61K 47/48, C08F 220/12

(54) **WIRKSTOFFHALTIGE POLYMERTEILCHEN**
POLYMER PARTICLES CONTAINING ACTIVE AGENTS
PARTICULES POLYMERES CONTENANT DES AGENTS ACTIFS

(30) Priorität: 05.04.2004 DE 102004016685
(43) Veröffentlichungstag der Anmeldung: 27.12.2006
(73) Patentinhaber: BASF Aktiengesellschaft, 67056 Ludwigshafen (DE)
(72) Erfinder: SIOL, Werner, 64297 Darmstadt (DE)
(86) Internationale Anmeldenummer: PCT/EP2005/003493
(87) Internationale Veröffentlichungsnummer: WO 2005/097197

(56) Entgegenhaltungen:
- EP-B- 0 808 174
- DE-A1- 1 944 693
- DE-A1- 4 328 069
- US-A- 4 225 581
- US-A- 5 985 573

## Beschreibung

### Gebiet der Erfindung

Die Erfindung betrifft wirkstoffhaltige Polymerteilchen im Größenbereich von 20nm bis 8µm, die in einem Teil des Bereichs von pH 0-10 wasserunlöslich und in einem anderen Teilbereich löslich sind.

### Stand der Technik

Die Synthese von Kunststoffteilchen durch Emulsionspolymerisation hat einen hohen Stand erreicht. So werden Polymerteilchen mit aktivierten Oberflächen in der Immundiagnostik eingesetzt (z.B. DE 31 16 995). Besonders einheitliche, 10µm große Teilchen als Eichstandards sind das erste kommerziell im Weltraum hergestellte Produkt (siehe Vanderhoff et al. US 5 106 903), Ugelstad beschreibt niedermolekulare Kunststoffteilchen aus beispielsweise PVC oder Polystyrol, die das 500fache ihres Eigenvolumens an Quellmitteln aufnehmen können (DE 2751867). Diese Teilchen können auch mit Wirkstoffen, z.B. Pflanzenschutzmitteln angequollen werden.
Impfstoffe auf der Basis von Styrol/ Acrylatteilchen werden in US 4225581 beschrieben. Mit speziellen Nanopartikeln läßt sich sogar die Blut- Hirn- Schranke überwinden (US 6 117 454).
Nachteil dieser Wirkstoffteilchen ist der Umstand, daß die Partikelträger nicht resorbierbar sind. Eine Ausnahme wird in einer neueren Patentanmeldung berichtet (P 103 53 989.1, noch unveröffentlicht). Darin wird berichtet, daß Kunststoffpartikel im Größenbereich 0,01- 20µm, die spezielle Oligoester der Glykolsäure und Milchsäure mit (Meth)acrylatendgruppen enthalten, den Anforderungen an bioresorbierbare Wirkstoffträger genügen.
Derartige Teilchen sind zunächst in Wasser unlöslich. Nach Hydrolyse beispielsweise der Milchsäureestergruppen zerfallen diese Teilchen jedoch in wasserlösliche Bestandteile.
Besonderes Interesse haben Emulsionspolymerisate als Überzugsmittel für Arzneimittel gefunden (DE 2135073). Je nachdem, ob diese Emulsionspolymerisate Carboxylgruppen oder Aminogruppen enthalten, lassen sich damit magensaftresistente, darmsaftlösliche oder magensaftlösliche Dragierlacke realisieren.
Dabei werden die Emulsionspolymerisate direkt oder als redispergierbare Pulver eingesetzt (DE 3208791).
Inzwischen finden derartige, funktionelle Acrylatdispersionen auch als dermales therapeutisches System Anwendung (DE 4310012). Auch werden Mikropartikel aus einem Koagulat aus Polymerdispersion und Wirkstoff beschrieben (DE 4328069)

### Aufgabe und Lösung

Während aminogruppenhaltige (d.h. magensaftlösliche) oder carboxylgruppenhaltige (d.h. darmsaftlösliche) Emulsionspolymerisate als Dragierfilm (also im Teilchenverbund) breite Anwendung finden, kommen diese Emulsionspolymerisate als wässerige Dispersion, d.h. als Einzelteilchen, als Wirkstoffträger im allgemeinen nicht zum Einsatz. Hier setzt man eher auf Liposomen als Träger für Arzneimittel.
Nach wie vor fehlt es an einer einfachen technischen Lösung zur gezielten Freisetzung von flüssigen Wirkstoffen oder Dispersionen.
Es wurde nun gefunden, dass wirkstoffhaltige Polymerteilchen in einem Größenbereich von 20nm bis 8µm, enthaltend 3 bis 1000 Teile Wirkstoff und 1 Teil Vinylpolymerisat aus 10- 80 Gew.% Amino- und/oder Carboxylgruppen enthaltenden Monomeren, das in einem Teil des pH- Bereichs von 0-10 unlöslich ist und in einem anderen Teilbereich löslich ist, das zu > 50 Gew% aus Polymeren mit einem Molekulargewicht < 100.000 Dalton besteht, bestens als partikuläre Wirkstoffträger geeignet sind. Dabei sind Vinylpolymere mit einem Molekulargewicht < 20.000 oder < 5.000 besonders bevorzugt.
Technisch interessant sind speziell solche wirkstoffhaltigen Polymerteilchen, die zu >60Gew% oder gänzlich aus den oben genannten Vinylpolymeren und Wirkstoffen aufgebaut sind.
Bevorzugt sind insbesondere wirkstoffhaltige Polymerteilchen deren Polymerkomponente aus
A) 20- 90 Gew% Alkylester der Acryl- und/oder Methacrylsäure,
B) 80- 10 Gew.% Monomeren mit Carboxylgruppen und/oder Aminogruppen,
C) 0- 40 Gew% weiteren, mit A) und B) copolymerisierbaren Monomeren besteht.
Als A) Alkylester sind Ester mit 1- 8 C-Atomen im Alkylrest, insbesondere
Methyl- und Ethylacrylat und - methacrylat zu nennen.
Geeignete Säuremonomere B) sind Acrylsäure und insbesondere Methacrylsäure. Weitere Säuremonomere sind Malein- Fumar- und Itakonsäure und Halbester dieser Säuren.
Geeignete Monomere B) mit Aminogruppen sind z. B. Vinylimidazol, Monoalkylamino- bzw. Dialkylaminoalkylester oder -alkylamide von polymerisier-baren Carbonsäuren, z.B. Dimethylaminoethylmethacrylat.
Als Monomere C) kommen ganz allgemein Vinylmonomere in Betracht, z.B. Hydroxyethylmethacrylat oder Styrol.
Im allgemeinen werden die Polymerteilchen entweder nur basische oder nur saure Monomere B) enthalten.
Das Mengenverhältnis der Monomeren A), B) und C) richtet sich nach den Erfordernissen der Freisetzung der Wirkstoffe.

Besonderes Interesse finden wirkstoffhaltige Teilchen, deren Polymerkomponenten nur aus Monomeren A) und B) bestehen.
Interessant sind z.B. Polymerkomponenten aus 50 Gew% Ethylacrylat und 50 Gew% Methacrylsäure. Besonders interessant sind wirkstoffhaltige Polymerteilchen, deren Polymerkomponente nur aus Methacrylatmonomeren besteht, z.B. aus 40- 80 Gew% Methylmethacrylat und 60- 20Gew% Methacrylsäure.
Es sei hier bereits darauf hingewiesen, dass das Löslichkeitsverhalten der mit Wirkstoff beladenen Polymerteilchen ganz wesentlich von der Hydrophobie des Wirkstoffs und seiner funktionellen Gruppen abhängt. Dies ergibt sich bereits einfach aus dem Mengenverhältnis Wirkstoff/ Polymerteilchen.

Im allgemeinen bestehen diese wirkstoffhaltigen Polymerteilchen aus 1 Teil pH- sensitives Polymer und 3-1000 Teilen Wirkstoff. D.h. die hier zum Einsatz kommenden Polymerteilchen können bis zum 1.000fachen ihres Eigengewichtes an Wirkstoff aufnehmen. Besonders interessant ist ein Gewichtsverhältnis von Polymerteilchen zu Wirkstoff im Bereich 1: 3 bis 1: 500, wobei der Bereich 1: 5 bis 1: 300 und insbesondere 1: 10 bis 1: 200 bevorzugt ist.
Bei flüssigen Wirkstoffen stellt der Wirkstoff ein Lösungsmittel oder Weichmachungsmittel für die Polymerteilchen dar. Dies führt dazu, dass sich in bestimmten Fällen die wirkstoffhaltigen Teilchen bei einer pH- Änderung schneller im Wasser lösen als die Polymerteilchen ohne Wirkstoff.

### Durchführung der Erfindung

Die Synthese der Polymerteilchen erfolgt im allgemeinen durch Emulsionspolymerisation nach dem Zulaufverfahren, wie sie beispielsweise in DE 2135073 beschrieben wird.
Dabei wird die Größe der Teilchen am einfachsten durch die Menge des vorgelegten Emulgators gesteuert.
Auf diesem Wege sind Polymerteilchen im Bereich 20nm- 500nm zugänglich.
Größere Teilchen lassen sich nach dem Saatlatexverfahren realisieren (siehe Beispiel 2).
Carboxylgruppenhaltige Emulsionspolymerisate werden in der Regel mit anionischen Emulgatoren, wie z.B. Natriumlaurylsulfat, hergestellt, aminogruppenhaltige Polymere mit kationischen oder nichtionischen Tensiden, wie z.B. oxethylierten Fettalkoholen.
Im allgemeinen wird die Polymerisation unter Inertgas z.B. Stickstoff durchgeführt.
Als Initiatoren kommen die bei Emulsionspolymerisationen verwendeten Systeme zum Einsatz, wie Ammoniumperoxodisulfat oder das Natriumsalz der 4,4'-Dicyano-4,4'-azovaleriansäure. Will man das Molekulargewicht der Polymeren durch die Menge des eingesetzten Initiators steuern, so können auch org. Peroxide wie z.B. t.-Butylperpivalat im Zulauf mitverwendet werden.

Üblicherweise wird das Molekulargewicht der Polymeren jedoch mit Hilfe von Polymerisationsreglern wie Mercaptanen eingestellt. Hier sind Alkanthiole und insbesondere Ester der Thioglykolsäure oder Mercaptopropionsäure zu nennen,
z.B. 2- Ethylhexylthioglykolat in Anteilen von 0,1- 10 Gew%, bevorzugt 0,3- 5 Gew%, bezogen auf den Polymerisatfeststoff.
Insbesondere beim Einsatz grösserer Anteile an Mercaptanen empfiehlt sich nach Abschluß der Polymerisation eine Desodorierung durch Entgasung bei reduziertem Druck.
Die Regelung des Molekulargewichtes der Polymeren ist für die Aufnahme großer Wirkstoffmengen von Bedeutung. Aus diesem Grunde sollte wenigstens ein Teil des Polymeren (z.B. wenigstens 50Gew% oder besser > 90Gew% ein Molekulargewicht < 100.000 Dalton aufweisen. Besser ist es, wenn das Molekulargewicht (Mw) < 30.000 oder bevorzugt < 20.000 Dalton ist. Besondere Vorteile erzielt man, wenn das Molekulargewicht im Bereich 1.000 bis 10.000 Dalton und besonders bevorzugt im Bereich 2.000 bis 8.000 Dalton liegt.
Interessanterweise bilden diese kurzkettigen Polymeren in Kombination mit den Wirkstoffen sehr stabile Teilchen, die beispielsweise als carboxylgruppenhaltige Teilchen im sauren Bereich, z.B. pH 2- 3, sehr stabil sind, während sie im neutralen bis alkalischen Bereich innerhalb von Sekunden oder Bruchteilen von Sekunden (je nach Größe und Wirkstoffgehalt der Teilchen) zerfallen.
U.U. ist die hohe Stabilität der wirkstoffhaltigen Polymerteilchen im unlöslichen pH-Bereich eine Frage der Osmose, die die Wirkstoffe als eine Art Lösungs- oder Verdünnungsmittel geradezu in die Teilchen zwingt.
Einmal gebildet, sind die Wirkstoffteilchen, z.B. als wässerige Dispersionen, bei gegebenem pH über Monate stabil. Sollten die Teilchen aufgrund ihrer Größe und einer von der wässerigen Phase abweichenden Dichte sedimentieren oder aufrahmen, können diese wirkstoffhaltigen Polymerteilchen durch kurzes Schütteln wieder dispergiert werden.
Hinsichtlich der Auswahl der Wirkstoffe gibt es kaum Begrenzungen. Unter Wirkstoffen sind im weitesten Sinne Arzneistoffe, Kosmetikwirkstoffe, UV- Schutzmittel, Parfümöle, Tierarzneimittel, ganz allgemein physiologische Wirkung entfaltende Stoffe zu verstehen. Insbesondere kommen als geeignete Wirkstoffe flüssige oder ölige Stoffe mit geringer Wasserlöslichkeit infrage.Besonderes Interesse verdienen Wirkstoffe, die eine Löslichkeit <50g/ L oder bevorzugt <10g/ L Wasser aufweisen. Feststoffe können bei erhöhtrer Temperatur oder in Gegenwart von Lösungsmitteln, z.B. Butylacetat, eingearbeitet werden.
In der Regel wird das Lösungsmittel nach erfolgter Einarbeitung des Wirkstoffs in die Polymerteilchen wieder entfernt (z.B. abdestilliert).

Besonders bei Wirkstoffen mit Amino- oder Säuregruppen ist darauf zu achten, dass die Polymerteilchen durch den Wirkstoff nicht in einen pH- Bereich gelangen, in dem sie sich auflösen. Nur mit Einschränkungen als Wirkstoffe geeignet sind hoch schmelzende Stoffe mit geringer Löslichkeit in organischen Lösungsmitteln. Gegebenenfalls müssen die Teilchen durch zudosierten Emulgator stabilisiert werden. Im allgemeinen ist jedoch ein Zusatz von Emulgator nicht oder nur für die Einarbeitung der Wirkstoffe erforderlich.
Bei sehr hohen Wirkstoff/Polymer- Verhältnissen kann es von Vorteil sein, die Wasserphase durch geringe Mengen Puffer, z.B. im ppm-Bereich, in dem für die Teilchen unlöslichen pH- Bereich, bei carboxylgruppenhaltigen Polymerteilchen z. B. im Bereich pH 3- 4 zu puffern. In der Regel wird man jedoch keinen Puffer einsetzen.
Das Einarbeiten der Wirkstoffe in die Polymerteilchen erfolgt im allgemeinen in wässeriger Dispersion.
Bei erhöhter Temperatur erfolgt die Einarbeitung bevorzugt in Rührapparaturen.
Das Einarbeiten von Flüssigkeiten bei Raumtemperatur kann durch einfaches Schütteln oder bevorzugt in Über-Kopf-Mischem erfolgen. Im allgemeinen wird nur langsam gerührt. Die Anwendung von großen Scherkräften (Turbomischer etc.) ist zu vermeiden.
Es sei nochmals darauf hingewiesen, daß die erfindungsgemäßen pH-sensitiven Polymer/ Wirkstoffkombinationen sehr stabil sind. So wird der Wirkstoff gewissermassen als osmotisches Verdünnungsmittel von den Polymerteilchen regelrecht aufgesogen. Aus diesem Grunde können auch sehr feinteilige wirkstoffhaltige Polymerteilchen (Durchmesser z. B. 50nm) realisiert werden, die durch einfaches Zerteilen auch bei hohen Scherkräften ansonsten nicht zugänglich sind.
In der Regel wird man die wirkstoffhaltigen Polymerteilchen direkt als wässerige Dispersionen einsetzen. Insbesondere bei festen Wirkstoffen ist es jedoch auch möglich, die wirkstoffhaltigen Polymerdispersionen beispielsweise zu gefriertrocknen, um so die feinen wirkstoffhaltigen Polymerteilchen als Feststoff zu gewinnen, der dann in den verschiedenartigsten Rezepturen zur besonders schnellen Freisetzung von Wirkstoffen verwendet werden kann. In der Regel werden die wirkstoffhaltigen Polymerteilchen jedoch mit 0,25- 999 Teilen Wasser pro Teil wirkstoffhaltiger Polymerteilchen eingesetzt, d.h. als wässerige Dispersion mit einem Wassergehalt im Bereich 20- 99,9 Gew%, bevorzugt im Bereich 40- 95 Gew%. Gegebenenfalls werden Konservierungsstoffe wie z.B. Ethyl-p-hydroxybenzoat zugesetzt.
Wenn es auch einfach möglich ist, eine gegebenenfalls sedimentierte oder aufgerahmte wirkstoffhaltige Polymerdispersion durch Schütteln wieder zu homogenisieren, können auch von vornherein Massnahmen getroffen werden, das Sedimentieren oder Aufrahmen der Teilchen zu verhindern, z.B. durch Anpassen der Dichte der wirkstoffhaltigen Polymerteilchen an die Dichte der wässerigen Phase oder aber durch Erhöhen der Viskosität der Wasserphase durch wasserlösliche Verdickungsmittel.

Die Größe der wirkstoffhaltigen Polymerteilchen wird in erster Linie durch die Größe der Polymerteilchen und das Wirkstoff/ Polymer- Verhältnis bestimmt. So wird ein 100nm großes Polymerteilchen, das die 7fache Menge an Wirkstoff aufnimmt, seine Masse ver 8- fachen. Dies bedeutet bei vergleichbarer Dichte von Wirkstoff und Polymerteilchen eine Verdopplung des Durchmessers auf 200nm. Entsprechend wird ein Teilchen (Durchmesser 100nm), das die 124fache Menge an Wirkstoff aufnimmt, entsprechend einem Anstieg der Gesamtmasse auf das 125fache, auf einen Durchmesser von 500nm vergrößert (siehe Beispiel 2).
Wie ausgeführt, können die pH- sensitiven, geregelten Polymerteilchen sogar das 1000fache ihres eigenen Volumens an Wirkstoff aufnehmen entsprechend einem Anstieg der Teilchengröße auf das 10fache.
Prinzipiell sind damit wirkstoffhaltige Polymerteilchen im Bereich 0,02- 20µm zugänglich, wobei der Größenbereich 0,04- 12µm bevorzugt und der Bereich
0,05- 8µm besonders bevorzugt ist. Besonders günstige Wirkstoff/ Polymerverhältnisse lassen sich mit wirkstoffhaltigen Polymerteilchen im Bereich >2- < 8µm erzielen.
In der Regel sind die wirkstoffhaltigen Polymerteilchen kugelförmig mit einer glatten Oberfläche.Bevorzugt handelt es sich um nicht koagulierte, frei bewegliche Einzelteilchen, in denen der Wirkstoff homogen verteilt ist. Bevorzugt sind die Teilchen monodispers, d.h. >80Gew% aller Teilchen zeigen denselben Teilchendurchmesser.Daneben ist es auch möglich, wirkstoffhaltige Polymerteilchen mit einer bimodalen oder multimodalen Teilchengrößenverteilung einzusetzen. Dies ist einerseits von Interesse, wenn man die Teilchen in wässeriger Dispersion mit einem-möglichst geringen Gehalt an Wasser zur Anwendung bringen möchte, andererseits bietet sich diese Möglichkeit an, wenn man in einer Dispersion unterschiedliche wirkstoffhaltige Polymerteilchen mit z.B. unterschiedlichen Freisetzungsbedingungen realisieren möchte.

Im allgemeinen ergibt sich mit diesen wirkstoffhaltigen Polymerteilchen bei Erreichen des entsprechenden pH- Wertes, bei dem das Teilchen löslich ist, eine schnelle Freisetzung des Wirkstoffes.
Eine langsame Freisetzung der Wirkstoffe mit fortschreitender Hydrolyse von vernetzenden oder hydrophoben Gruppen, wie sie in der unveröffentlichten Patentanmeldung P 10353989.1 beschrieben wird, ist nicht erfindungsgemäß.
D.h. die erfindungsgemäßen wirkstoffhaltigen Polymerteilchen sind auch dadurch gekennzeichnet, dass die Polymerteilchen einen Gehalt an < 1 Gew% an Monomeren der allgemeinen Formel

(CH₂=CR₁-CO-(-O-CHR₂-CO-)ₘ-O-)ₙ-R₃ (I)

wobei R₁ und R₂ unabhängig von einander für H oder CH₃ stehen,
m für 1- 20 steht und
R₃ für einen gegebenenfalls substituierten Alkylrest mit 1-18 Kohlenstoffatomen für n= 1 bzw. für einen gegebenenfalls substiuierten Alkylidenrest mit 2- 18
Kohlenstoffatomen für n=2 steht, aufweisen.

Ganz besonders bevorzugt sind wirkstoffhaltige Polymerteilchen, deren Polymerkomponente die oben genannten Monomeren (I) überhaupt nicht enthalten.

Besondere Vorteile der neuen wirkstoffhaltigen Polymerteilchen

Von besonderer Bedeutung für eine breite Anwendung der wirkstoffhaltigen Polymerpartikel ist der Umstand, dass die chemische Zusammensetzung der diese Wirkstoffteilchen aufbauenden Polymeren seit Jahrzehnten als Dragierlack in pharmazeutischen Rezepturen bekannt ist. So ist der Weg dieser Polymeren im Körper bestens untersucht. Die hier angewandten Polymeren unterscheiden sich von den als Dragierlack eingesetzten Produkten im wesentlichen im Molekulargewicht. In den wirkstoffhaltigen Kunststoffpartikeln kommen kürzere Polymere zum Einsatz. Erfahrungsgemäß kann man von diesen kürzeren Ketten einen noch besseren Abbau erwarten.
Hinzu kommt, dass aufgrund der hohen Aufnahmefähigkeit der Polymerpartikel für den Wirkstoff der Anteil an Polymer bezogen auf den Wirkstoff sehr gering ist, z.B. < 1 Gew% (siehe Beispiel 2).
Interessant ist auch, dass die wirkstoffhaltigen Kunststoffpartikel in ihrem Ausgangs pH- Bereich, z.B. pH 3 bei carboxylgruppenhaltigen Polymerteilchen sehr stabil sind, während sie beim Wechsel auf pH 7 den Wirkstoff praktisch augenblicklich frei geben.

Prinzipiell ist es möglich, 2 verschiedene wirkstoffhaltige Polymerpartikel mit 2 sich störend beeinflussenden Wirkstoffen in derselben, wässerigen Dispersion anzuwenden, wenn diese Wirkstoffe in Wasser unlöslich sind und die Dispersionen nur geringe Mengen an Emulgator enthalten. Selbstverständlich muß die Herstellung der beiden wirkstoffhaltigen Polymerteilchenarten getrennt erfolgen
Von Bedeutung ist auch, dass die Herstellung der wirkstoffhaltigen Polymerpartikel auch mit sehr empfindlichen Wirkstoffen durchgeführt werden kann, da die Einarbeitung der Wirkstoffe in die Polymerteilchen in der Regel durch einfaches Schütteln bei Raumtemperatur erfolgt.

Besonders hervorzuheben ist der Umstand, dass diese wirkstoffhaltigen Polymerpartikel bestens zur Verabreichung von Flüssigkeiten und Ölen geeignet sind. Einerseits läßt sich auf diesem Wege die Menge des Wirkstoffs durch die vorgegebene Verdünnung der Dispersion auf ein vorgegebene, einfach zu dosierende Menge (10Tropfen, 1 Meßlöffel etc) einstellen, andererseits ist erstmals eine einfache, alkoholfreie Verabreichung von wasserunlöslichen flüssigen Wirkstoffen und Essenzen möglich.
Die Erfindung beeinhaltet also auch ein Verfahren, das dadurch gekennzeichnet ist, dass 1 Teil Polymerisat einer wässerigen Polymerdispersion aus einem Vinylpolymerisat, das aus 10- 80 Gew% Amino- und/oder Carboxylgruppen enthaltenden Monomeren aufgebaut ist und das in einem Teil des pH- Bereichs von 0-10 unlöslich ist und in einem anderen Teilbereich löslich ist, das zu > 50 Gew% aus Polymeren mit einem Molekulargewicht < 100.000 Dalton besteht, mit 3-1000 Teilen Wirkstoff angequollen wird, wobei wirkstoffhaltige Polymerteilchen in einem Größenbereich von 20nm- 8µm gebildet werden und diese wirkstoffhaltigen Polymerpartikel appliziert werden.

Die Beispiele D1- D3 beschreiben die Synthese der pH- sensitiven Polymerdispersionen am Beisiel carboxylgruppenhaltiger Polymerer,
Beispiele 1- 4 beschreiben die Herstellung der wirkstoffhaltigen Polymerpartikel, Beispiel 5 beschreibt die Freisetzung des Wirkstoffs aus diesen Partikeln.

### Beispiel D1 Synthese einer feinteiligen, carboxylgruppenhaltigen Polymerdispersion mit einheitlicher Teilchengröße

In einem gerührten Reaktor werden 0,1g Natriumlaurylsulfat in 500g Wasser vorgelegt. Nach Zugabe von 50g einer 1 %igen Lösung von Kaliumperoxodisulfat in Wasser wird bei 80°C eine Mischung aus
105g Methylmethacrylat,
105g Methacrylsäure,
0,6g 2- Ethylhexylthioglykolat,
0,2g Natriumlaurylsulfat,
2g Wasser
innerhalb von 3h bei 80°C unter Argon zudosiert. Danach wird eine weitere h bei 80°c gerührt. Nach Filtration über ein feinmaschiges Siebgewebe wird eine feinteilige Dispersion erhalten. Feststoffgehalt: 27 %, pH. 3, Teilchendurchmesser 0,15µm.

### Beispiel D2 Synthese einer hoch quellbaren, carboxylgruppenhaltigen Polymerdispersion nach dem Saatlatexverfahren.

In einer Apparatur gemäß Beispiel D1 werden 20g der Dispersion D1 in 600g einer 1 %igen Lösung von Kaliumperoxodisulfat in Wasser vorgelegt. Hierzu dosiert man bei 75°C eine Mischung aus 85g Methylmethacrylat, 85g Methacrylsäure, 7g Butanthiol, 0,2g Natriumlaurylsulfat und 3,7g Wasser.
Nach Ende des Zulaufs wird eine auf 40°C erwärmte Lösung von 0,14g Kaliumperoxodisulfat und 0,12g Natriumlaurylsulfat in 40g Wasser zudosiert. Danach wird 1 h bei 80°C gerührt.
Nach Filtration erhält man eine Dispersion mit einem Feststoffgehalt von 21%, pH 3, Teilchengröße ca. 0,5µm. Die Polymerteilchen sind sehr einheitlich, sie sedimentieren, lassen sich aber einfach wieder aufschütteln. Das Molekulargewicht von > 90% der Polymeren ist < 10.000 Dalton.
Lösungsversuch bei Wechsel des pH:
Tropft man die Dispersion D2 in eine Phophatpufferlösung pH 7,0, so lösen sich die Teilchen innerhalb von 30s vollständig auf.

### Beispiel D3 Synthese einer feinteiligen, hoch quellbaren, carboxylgruppenhaltigen Polymerdispersion.

Entsprechend dem Verfahren gemäß Beispiel D1 wird eine Lösung von 0,24g Kaliumperoxodisulfat und 0,11g Natriumlaurylsulfat in 525g Wasser vorgelegt. Hierzu dosiert man bei 80°C 3g einer Mischung aus 74g Methylmethacrylat, 73g Methacrylsäure, 0,28g 2-Ethylhexylthioglykolat, 0,2g Natriumlaurylsulfat und 2g Wasser. Danach werden 6,5g Butanthiol dem Rest der Mischung zugegeben und diese Mischung innerhalb von 2h in die Vorlage dosiert.
Schließlich wird der Ansatz bei reduziertem Druck (p= 500mbar) desodoriert.
Man erhält eine feinteilige Dispersion, Feststoff: 23,4 %, pH 3,
Teilchengröße ca. 0,1 µm.
Beim Eintropfen in Phospatpuffer pH 7,0 lösen sich die Teilchen in 1s auf.

### Einarbeitung von 2-Phenylpropanol (1) als Modellwirkstoff:

### Beispiel 1 Wirkstoff/ Polymerverhältnis= 14,3/1

2,0g Dispersion D2 (enthaltend 0,42gPolymer),0,1g Natriumlaurylsulfat, 40g Wasser und 6,0g 2.Phenylpropanol (1) werden in einer 50ml Laborflasche 4h über Kopf gedreht. Man erhält eine stabile Dispersion mit einer Teilchengröße von ca. 1,2µm.

### Beispiel 2 Wirkstoff/ Polymer- Verhältnis= 128,6/1

1,0g der wirkstoffhaltigen Dispersion gemäß Beispiel 1, enthaltend 0,125 g 2-Phenylpropanol (1) und 0,00875g Polymer, werden mit 10g Wasser und 1,0g 2-Phenylpropanol (1) 4h über Kopf gedreht. Man erhält eine stabile Dispersion mit einer Teilchengröße von 2,5µm (einheitlich).
Nach 14 Tagen Stehen bei RT ist die Dispersion sedimentiert. Durch kurzes Schütteln erhält man wieder eine homogene Dispersion.

### Beispiel 3 Wirkstoff/ Polymer- Verhältnis= 40,8/1

0,109g der Dispersion gemäß D3 werden mit 5mg Natriumlaurylsulfat in 7,8g Wasser und 1,016g 2-Phenylpropanol (1) 2h über Kopf gedreht. Man erhält eine stabile, feinteilige wirkstoffhaltige Polymerdispersion.

### Beispiel 4 Wirkstoff/ Polymer- Verhältnis= 3/ 1

1,2g Dispersion D1 werden mit 10mg Natriumlaurylsulfat, 7,0g Wasser und 1,0g 2-Phenylpropanol (1) über Kopf gedreht. Man erhält eine stabile, feinteilige Dispersion.

### Beispiel 5 Freisetzung des Wirkstoffs durch pH- Änderung

Man tropft die 1,2 µm großen wirkstoffhaltigen Polymerteilchen gemäß Beispiel 1 in eine Phosphatpufferlösung pH 7,0. Die Teilchen lösen sich innerhalb von 1 s.
Man wiederholt den Versuch mit den 2,5µm großen wirkstoffhaltigen Polymerteilchen gemäß Beispiel 2. Auch diese Teilchen lösen sich bei pH 7,0 innerhalb von 1 s.

## Patentansprüche

1. Wirkstoffhaltige Polymerteilchen, enthaltend ein Vinylpolymerisat aus 10- 80 Gew% Amino- und/oder Carboxylgruppen enthaltenden Monomeren, das in einem Teil des pH- Bereichs von 0-10 unlöslich ist und in einem anderen Teilbereich löslich ist, **dadurch gekennzeichnet, dass** diese wirkstoffhaltigen Polymerteilchen
- pro Teil Vinylpolymerisat 3-1000 Teile Wirkstoff enthalten,
- eine Teilchengröße im Bereich 20nm - 8µm aufweisen und
- das Vinylpolymerisat zu > 50 Gew% aus Polymeren mit einem Molekulargewicht < 100.000 Dalton aufgebaut ist.

2. Wirkstoffhaltige Polymerteilchen gemäß Anspruch 1, **dadurch gekennzeichnet, dass** diese Teilchen in Form einer wässerigen Dispersion mit einem Wassergehalt von 20- 99,9 Gew% vorliegen.

3. Wirkstoffhaltige Polymerteilchen gemäß den Ansprüchen 1 und 2,
**dadurch gekennzeichnet, dass** die Polymerkomponente aus
A) 20- 90 Gew.% Alkylestern der Acryl- und/oder Methacrylsäure,
B) 80-10 Gew.% Monomeren mit Carboxylgruppen und/oder Aminogruppen,
C) 0- 40 Gew. % weiteren, mit A) und B) copolymerisierbaren Monomeren besteht.

4. Wirkstoffhaltige Polymerteilchen gemäß den Ansprüchen 1- 3, **dadurch gekennzeichnet, dass** die Polymerkomponente zu > 90 Gew.% aus Polymeren mit einem Molekulargewicht < 20.000 Dalton aufgebaut ist.

5. Wirkstoffhaltige Polymerteilchen gemäß den Ansprüchen 1- 4, **dadurch gekennzeichnet, dass** als Wirkstoffe flüssige oder ölige Substanzen mit einer Wasserlöslichkeit < 10g / L zum Einsatz kommen.

6. Verfahren zur Applikation von Wirkstoffen, **dadurch gekennzeichnet, dass** eine wässerige Polymerdispersion enthaltend ein Vinylpolymerisat, das aus 10- 80 Gew.% Amino- und/oder Carboxylgruppen enthaltenden Monomeren aufgebaut ist und das in einem Teil des pH- Bereichs von 0-10 unlöslich und in einem anderen löslich ist, das zu > 50 Gew.% aus Polymeren mit einem Molekulargewicht < 100.000 Dalton besteht, in einem pH- Bereich, in dem das Polymere unlöslich ist, mit 3- 1000 Teilen Wirkstoff pro Teil Polymer angequollen wird, wobei wirkstoffhaltige Polymerteilchen in einem Größenbereich von 20nm- 8µm gebildet werden, die dann als wirkstoffhaltige Polymerpartikel appliziert werden.

## Claims

1. Active ingredient-containing polymer particles comprising a vinyl polymer from 10-80% by weight monomers comprising amino and/or carboxyl groups, which is insoluble in one part of the pH range 0-10 and is soluble in another part of the range, wherein these active ingredient-containing polymer particles
- comprise 3-1000 parts of active ingredient per part of vinyl polymer,
- have a particle size in the range 20 nm-8 µm and
- the vinyl polymer is > 50% by weight composed of polymers having a molecular weight of < 100 000 daltons.

2. Active ingredient-containing polymer particles according to claim 1, which particles are in the form of an aqueous dispersion having a water content of 20-99.9% by weight.

3. Active ingredient-containing polymer particles according to claims 1 and 2, wherein the polymer component consists of
A) 20-90% by weight alkyl esters of acrylic and/or methacrylic acid,
B) 80-10% by weight monomers having carboxyl groups and/or amino groups,
C) 0-40% by weight further monomers copolymerizable with A) and B).

4. Active ingredient-containing polymer particles according to claims 1-3, wherein the polymer component is > 90% by weight composed of polymers having a molecular weight of < 20 000 daltons.

5. Active ingredient-containing polymer particles according to claims 1-4, wherein liquid or oily substances having a solubility of < 10 g/l of water are employed as active ingredients.

6. A method for administering active ingredients, which comprises swelling an aqueous polymer dispersion comprising a vinyl polymer which is composed of 10-80% by weight monomers comprising amino and/or carboxyl groups and which is insoluble in one part of the pH range 0-10 and is soluble in another and which is > 50% by weight composed of polymers having a molecular weight of < 100 000 daltons, in a pH range in which the polymer is insoluble, with 3-1000 parts of active ingredient per part of polymer, with formation of active ingredient-containing polymer particles in a size range of 20 nm-8 µm, and administering these active ingredient-containing polymer particles.

## Revendications

1. Particules polymères contenant des agents actifs, contenant un polymère vinylique à base de 10 à 80% en poids de monomères contenant des groupes amino et/ou carboxyle, qui est insoluble dans une partie de la plage de pH de 0 à 10 et est soluble dans une autre plage partielle, **caractérisées en ce que** ces particules polymères contenant des agents actifs :
- contiennent, par partie de polymère vinylique, de 3 à 1000 parties d'agent actif,
- présentent une taille de particules de l'ordre de 2 0 nm à 8 µm et
- le polymère vinylique est constitué à plus de 50% en poids de polymères d'un poids moléculaire de moins de 100 000 Dalton ;

2. Particules polymères contenant des agents actifs suivant la revendication 1, **caractérisées en ce que** ces particules se présentent sous la forme d'une dispersion aqueuse d'une teneur en eau de 20 à 99,9% en poids.

3. Particules polymères contenant des agents actifs suivant les revendications 1 et 2, **caractérisées en ce que** le composant polymère consiste en :
A) de 20 à 90% en poids d'esters alkyliques de l'acide acrylique et/ou méthacrylique,
B) de 80 à 10% en poids de monomères possédant des groupes carboxyle et/ou des groupes amino,
C) de 0 à 40% d'autres monomères copolymérisables avec A) et B).

4. Particules polymères contenant des agents actifs suivant les revendications 1 à 3, **caractérisées en ce que** le composant polymère est constitué à plus de 90% en poids de polymères d'un poids moléculaire de moins de 20 000 Dalton.

5. Particules polymères contenant des agents actifs suivant les revendications 1 à 4, **caractérisées en ce que** l'on met en oeuvre en tant qu'agents actifs des substances liquides ou huileuses présentant une solubilité dans l'eau de moins de 10 g/1.

6. Procédé d'application d'agents actifs, **caractérisé en ce qu'**une dispersion aqueuse de polymère contenant un polymère vinylique constitué de 10 à 80% en poids de monomères contenant des groupes amino et/ou carboxyle et qui est insoluble dans une partie de la plage de pH de 0 à 10 et est soluble dans une autre partie de plage, qui est constitué à plus de 50% en poids de polymères d'un poids moléculaire de moins de 100 000 Dalton, est gonflée avec 3 à 1000 parties d'agent actif par partie de polymère dans une plage de pH dans laquelle le polymère est insoluble, et selon lequel il se forme des particules de polymère contenant des agents actifs d'un ordre de grandeur de 20 nm à 8 µm, qui sont alors appliquées en tant que particules polymères contenant des agents actifs.
